# EUROPEAN PATENT APPLICATION

(11) **EP 4 295 848 A1**
(43) Date of publication of application: **27.12.2023**
(21) Application number: 22756488.7
(22) Date of filing: 16.02.2022
(51) Int. Cl.: A61K 31/4545, A61K 31/4525, A61K 33/243, A61K 45/06, A61P 35/00

(54) **COMPOSITION FOR TREATMENT OF ANTICANCER DRUG RESISTANCE**

(30) Priority: 17.02.2021 KR 20210021215
(71) Applicant: Industry-Academic Cooperation Foundation, Yonsei University, Seoul 03722 (KR)
(72) Inventor: CHEONG, Jae-Ho, Seoul 06628 (KR); KIM, Jae-woo, Seoul 06574 (KR); YOON, Bo Kyung, Seoul 04731 (KR)
(74) Representative: Pharma Patents International AG
(86) International application number: PCT/KR2022/002290
(87) International publication number: WO 2022/177290

(57) **Abstract**

A composition according to the present invention, when used alone, is capable of inhibiting the growth of anticancer drug-resistant cancer by overcoming anticancer drug resistance or enhancing anticancer drug sensitivity, and when co-administered with an anticancer drug, may be used very effectively for the prevention, alleviation or treatment of various cancers, especially anticancer drug-resistant cancer.

## Description

### Technical Field

The present invention relates to a composition capable of enhancing anticancer drug sensitivity, thereby overcoming anticancer drug resistance or inhibiting the growth of anticancer drug-resistant cancer.

### Background Art

Cancer is a very fatal disease that can threaten the life of an individual by causing cells constituting tissues to proliferate abnormally and indefinitely to form tumors, thereby making organs unable to perform their normal functions. In 2017, the first leading cause of death in Korea was malignant neoplasm (cancer), and 27.6% of all deaths were due to cancer. In particular, gastric cancer (GC) is the third most common lethal cancer in the world, and although there are differences in the cancer site depending on race, gender, and region, molecular genomic technology has recently made it possible to classify the types of gastric cancers according to molecular characteristics.

Among biologically related subtypes of gastric cancer, the type with stem-like properties, in particular, has malignant biological properties that are difficult to treat and has the worst prognosis due to non-response to standard treatment (chemotherapy), and as immune checkpoint blockade therapy has also been found to be ineffective against stem-like/EMT cancer subtypes, there is a problem in that targeted therapy cannot be applied to stem-like/EMT cancer subtypes. In addition, anticancer drugs that are currently used for gastric cancer patients include epirubicin, cisplatin, fluorouracil, etc. These anticancer drugs have a problem in that cancer types with stem-like properties show drug resistance, which reduces the successful cancer treatment effects of the anticancer drugs, thus worsening the prognosis of gastric cancer.

Accordingly, the present inventors have developed a combination drug for treating anticancer drug resistance that is capable of effectively treating malignant gastric cancer by overcoming anticancer drug resistance.

### DISCLOSURE

### Technical Problem

An object of the present invention is to provide a pharmaceutical composition for treating anticancer drug resistance and a method for treating anticancer drug resistance.

Another object of the present invention is to provide a pharmaceutical composition for enhancing anticancer drug sensitivity and a method for enhancing anticancer drug sensitivity.

Still another object of the present invention is to provide a pharmaceutical composition for preventing or treating anticancer drug-resistant cancer and a method for preventing or treating anticancer drug-resistant cancer.

Yet another object of the present invention is to provide a method for screening a composition for treating anticancer drug resistance.

However, objects to be achieved by the present invention are not limited to the above-mentioned objects, and other objects not mentioned herein will be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present invention pertains.

One embodiment of the present invention is directed to a pharmaceutical composition for overcoming anticancer drug resistance.

In the present invention, the composition may contain a myocardin-related transcription factor A (MRTF A) inhibitor as an active ingredient.

In the present invention, the MRTF A inhibitor may comprise a compound represented by Formula 1 below or a pharmaceutically acceptable salt thereof:

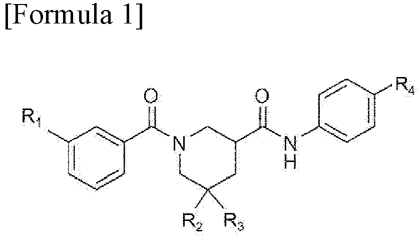

wherein
R₁ is a 5- or 6-membered ring heteroaryl, and R₂ to R₄ may each independently be hydrogen or halogen, wherein the heteroaryl may be furan or pyridine.

The MRTF A inhibitor of the present invention functions to inhibit Rho/MRTF/SRF signaling, and the compound represented by Formula 1 may be at least one selected from the group consisting of CCG-232601, CCG-203971, and CCG-222740, but is not limited thereto and may be any type of inhibitor that performs the same function.

In one embodiment of the present invention, CCG-232601 is a compound wherein R₁ in Formula 1 is pyridine, R₂ and R₃ are fluorine, and R₄ is chloride, and may be a compound represented by Formula 2 below, which is N-(4-chlorophenyl)-5,5-difluoro-1-(3-(pyridin-4-yl)benzoyl)piperidine-3-carboxamide. The CCG-232601 compound of the present invention is an inhibitor of the Rho/MRTF/SRF signaling pathway, is known as a potential anti-fibrosis therapeutic agent for systemic scleroderma, and is also known to suppress the development of bleomycin-induced dermal fibrosis when administered orally to mice.

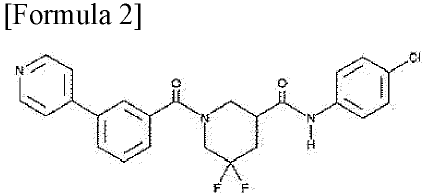

In another embodiment of the present invention, CCG-203971 is a compound wherein R₁ in Formula 1 is furan, R₂ and R₃ are hydrogen, and R₄ is chloride, and may be a compound represented by Formula 3 below, which is N-(4-chlorophenyl)-1-(3-(furan-2-yl)benzoyl)piperidine-3-carboxamide. The CCG-203971 compound of the present invention is an inhibitor of the Rho/MKL1/SRF transcriptional pathway, and is known to play a role in metastasis of melanoma and breast cancer and to be clinically associated with prostate cancer, and the Rho/MRTF/SRF pathway is also known to be involved in several types of fibrosis. It corresponds to a compound that represses both matrix stiffness and TGF-β-mediated fibrogenesis in human colonic myofibroblasts and shows antifibrotic activity in a murine model of skin injury and in lung fibrosis lung fibroblasts.

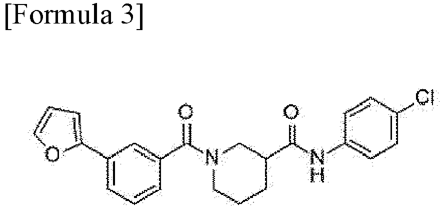

In another embodiment of the present invention, CCG-222740 is a compound wherein R₁ in Formula 1 is furan, R₂ and R₃ are fluorine, and R₄ is chloride, and may be a compound represented by Formula 4 below, which is N-(4-chlorophenyl)-5,5-difluoro-1-(3-(furan-2-yl)benzoyl)piperidine-3-carboxamide. The CCG-222740 compound of the present invention is an MRTF/SRF inhibitor, and corresponds to a substance known to prevent the expression of alpha smooth muscle actin protein and to effectively prevent scar tissue formation in a preclinical fibrosis model due to its less cytotoxicity.

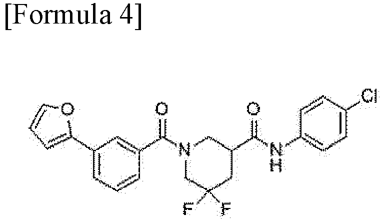

The compounds represented by Formulas 1 to 4 according to the present invention can treat anticancer drug resistance very effectively. The composition of the present invention can be used very effectively to prevent, alleviate or treat cancer by overcoming anticancer drug resistance of stem-like malignant cancer.

Examples of the pharmaceutically acceptable salt of the present invention include acid or base addition salts, and stereochemical isomers thereof. For example, the compounds may be in the form of organic or inorganic acid addition salts. Salts are those having desirable effects in patients when administered to the patients, and include, but are not particularly limited to, any salts that retain the activity of their parent compounds. These salts may include inorganic salts and organic salts, for example, salts of acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, lactic acid, bicarbonic acid, bisulfuric acid, bitartaric acid, oxalic acid, butyric acid, calcium edetate, carbonic acid, chlorobenzoic acid, citric acid, edetic acid, toluenesulfonic acid, fumaric acid, gluceptic acid, esilic acid, pamoic acid, gluconic acid, methyl nitric acid, malonic acid, hydrochloric acid, hydroiodoic acid, hydroxynaphtholic acid, isethionic acid, lactobionic acid, mandelic acid, mucous acid, napsylic acid, muconic acid, p-nitromethanesulfonic acid, hexamic acid, pantothenic acid, monohydrogen phosphoric acid, dihydrogen phosphoric acid, salicylic acid, sulfamic acid, sulfanilic acid, methanesulfonic acid, and the like. Base addition salts may include salts of alkali metal or alkaline earth metal salts, such as salts of ammonium, lithium, sodium, potassium, magnesium, calcium, and the like; salts with organic bases, such as salts of benzathine, N-methyl-D-glucamine, hydrabamine, and the like; and salts with amino acids such as arginine, lysine, and the like. In addition, these salts may be converted into a free form by treating with a proper base or acid.

In the present invention, the term "anticancer drug resistance" refers to a condition in which the effect of an anticancer drug decreases when the anticancer drug is used repeatedly in a fixed amount, or a condition in which the amount or frequency of use of an anticancer drug needs to be increased in order to obtain the same effect as previously experienced by a patient with anticancer drug resistance, or a condition in which the same effect as before is not obtained even when the same dose of a substance as before is administered.

In the present invention, the phrase "overcoming resistance (treating resistance)" refers to an action of recovering from a condition in which the effect of an anticancer drug decreases when the anticancer drug is used repeatedly in a fixed amount, or a condition in which the amount or frequency of use of an anticancer drug needs to be increased in order to obtain the same effect as previously experienced by a patient with anticancer drug resistance, or a condition in which the same effect as before is not obtained even when the same dose of a substance as before is administered. More specifically, the phrase "overcoming resistance (treating resistance)" refers to an action of making an anticancer drug exhibit the same anticancer effect even when the anticancer drug is applied less times or at lower doses, or an action of returning to a state prior to the occurrence of anticancer drug resistance so that the same effect can be obtained even when the same or lower dose of an anticancer drug as before is administered.

In the present invention, the anticancer drug that causes resistance as described above is a drug having a mechanism of killing cancer cells, which corresponds to a drug having an anticancer effect, and refers to a drug having a cancer treatment effect. Although the anticancer drug is not particularly limited in the kind thereof, it may comprise, for example, at least one selected from the group consisting of cisplatin, nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, navelbine, fadrazole, tamoxifen, toremifene, testolactone, anastrozole, letrozole, vorozol, bicalutamide, lomustine, and carmustine. Preferably, the anticancer drug may be a platinum-based anticancer drug, without being limited thereto.

In the present invention, the term "cancer" refers to or describes the physiological condition typically characterized by unregulated cell growth in a mammal. The cancer may be at least one selected from the group consisting of gastric cancer, thyroid cancer, parathyroid cancer, ovarian cancer, colorectal cancer, pancreatic cancer, liver cancer, breast cancer, cervical cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, and pituitary adenoma. Preferably, the cancer may be gastric cancer, but is not limited thereto and may be any type of cancer whose progression (such as tumor differentiation and/or proliferation) is dependent on the cancer cells or cancer stem cells described in the present invention.

In addition, the pharmaceutical composition for overcoming anticancer drug resistance according to the present invention may further contain an anticancer drug, thereby exhibiting a significantly improved effect of overcoming resistance. Here, although the anticancer drug that may be further contained is not particularly limited in the kind thereof, it may comprise, for example, at least one selected from the group consisting of cisplatin, nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, navelbine, fadrazole, tamoxifen, toremifene, testolactone, anastrozole, letrozole, vorozol, bicalutamide, lomustine, and carmustine. Preferably, the anticancer drug may be a platinum-based anticancer drug, and more preferably, may be at least one selected from the group consisting of cisplatin, carboplatin, and oxaliplatin.

Specifically, in the present invention, the compounds represented by Formulas 1 to 4, when co-administered with a general anticancer drug, more preferably a platinum-based anticancer drug, can overcome anticancer drug resistance, thereby providing a very high synergistic effect on inducing the inhibition of growth or apoptosis of cancer cells or cancer stem cells.

In the present invention, the term "co-administration" refers to a method of administering individual therapeutic components simultaneously, sequentially, or individually. Specifically, the term means obtaining the effect of combination therapy either by a method of administering two or more drugs simultaneously or sequentially, or by a method of alternately administering two or more drugs at regular or random intervals. The combination therapy may be defined as a method whose therapeutic effect measured based on the degree of response, the rate of response, the period until disease progression, or the survival period is better than the effect obtainable by administering each of the components for combination therapy alone at a conventional dose, and which may provide a synergistic effect, without being limited thereto.

In one embodiment of the present invention, the compound represented by any one of Formulas 1 to 4 and the anticancer drug may be contained at a weight ratio of 1:0.01 to 100 or 1:0.1 to 400, without being limited thereto, and the content ratio may be appropriately adjusted depending on the kind of anticancer drug used. In addition, the compounds represented by Formulas 1 to 4 may be administered before or simultaneously with the use of the anticancer drug to which resistance is to be overcome, and more preferably, may be administered 12 to 36 hours, most preferably 18 to 30 hours, before the use of the anticancer drug. If the content ratio is out of the above range, there may be problems in that the effect of overcoming anticancer drug resistance by co-administration cannot be obtained, and in that toxicity by the drug may occur in the subject of interest.

In another embodiment of the present invention, the compound represented by any one of Formulas 1 to 4 and the platinum-based anticancer drug may be contained at a weight ratio of 1:0.1 to 10, without being limited thereto. In addition, the compounds represented by Formulas 1 to 4 may be administered before or simultaneously with the use of the platinum-based anticancer drug to which resistance is to be overcome, and more preferably, may be administered 12 to 36 hours, most preferably 18 to 30 hours, before the use of the platinum-based anticancer drug. If the content ratio is out of the above range, there may be problems in that the effect of overcoming anticancer drug resistance by co-administration cannot be obtained, and in that toxicity by the drug may occur in the subject of interest.

In another embodiment of the present invention, the compound represented by any one of Formulas 1 to 4 and cisplatin may be contained at a weight ratio of 1:0.1 to 10, without being limited thereto. In addition, the compounds represented by Formulas 1 to 4 may be administered before or simultaneously with the use of cisplatin to which resistance is to be overcome, and more preferably, may be administered 12 to 36 hours, most preferably 18 to 30 hours, before the use of cisplatin. If the content ratio is out of the above range, there may be problems in that the effect of overcoming anticancer drug resistance by co-administration cannot be obtained, and in that toxicity by the drug may occur in the subject of interest.

In another embodiment of the present invention, the compound represented by any one of Formulas 1 to 4 and oxaliplatin may be contained at a weight ratio of 1:0.1 to 10, without being limited thereto. In addition, the compounds represented by Formulas 1 to 4 may be administered before or simultaneously with the use of oxaliplatin to which resistance is to be overcome, and more preferably, may be administered 12 to 36 hours, most preferably 18 to 30 hours, before the use of oxaliplatin. If the content ratio is out of the above range, there may be problems in that the effect of overcoming anticancer drug resistance by co-administration cannot be obtained, and in that toxicity by the drug may occur in the subject of interest.

In addition, the composition of the present invention may contain, as an active ingredient, an agent for reducing the activity or expression level of myocardin-related transcription factor A (MRTF A) protein, or an agent for reducing the expression level of a gene encoding the protein.

In the present invention, the agent for reducing the activity or expression level of the protein may comprise any one or more selected from the group consisting of compounds, peptides, peptide mimetics, aptamers, antibodies, and natural products, which bind specifically bind to the protein or a portion thereof, without being limited thereto. It corresponds to a means for providing an effect of inhibiting the activity or expression of the target MRTF A protein by acting directly or indirectly on the target MRTF A protein, without being limited thereto, and may be any agent that can be easily obtained by a known technique commonly used in the art.

In the present invention, the "peptide mimetics" are peptides or non-peptides that inhibit the binding domain of MRTF A protein, leading to the inhibition of MRTF A activity. Non-hydrolyzable peptide analogs of critical residues can be generated using β-turn dipeptide cores (Nagai et al. Tetrahedron Lett 26:647, 1985), keto-methylene pseudopeptides (Ewenson et al. J Med chem 29:295, 1986; and Ewenson et al. in Peptides: Structure and Function (Proceedings of the 9th American Peptide Symposium) Pierce chemiCal co. Rockland, IL, 1985), azepine (Huffman et al. in Peptides: chemistry and Biology, G.R. Marshall ed., EScOM Publisher: Leiden, Netherlands, 1988), benzodiazepine (Freidinger et al. in Peptides; chemistry and Biology, G.R. Marshall ed., EScOM Publisher: Leiden, Netherlands, 1988), β- aminoalcohols (Gordon et al. Biochem Biophys Res commun 126:419 1985), and substituted gamma lactam rings (Garvey et al. in Peptides: chemistry and Biology, G.R. Marshell ed., EScOM Publisher: Leiden, Netherlands, 1988).

In the present invention, the "aptamer" refers to a single-stranded nucleic acid (DNA, RNA or modified nucleic acid) characterized by having a stable own tertiary structure while capable of binding to a target molecule with high affinity and specificity. Since a technology of developing aptamers, called systematic evolution of ligands by exponential enrichment (SELEX), was first developed (Ellington, AD and Szostak, JW., Nature 346:818-822, 1990), many aptamers capable of binding to various target molecules, including small-molecular organic materials, peptides, and membrane proteins, have been continuously developed. Aptamers are comparable to single antibodies due to their intrinsic characteristics of binding to target molecules with high affinity (usually a pM level) and specificity, and in particular, have high potential as alternative antibodies to the extent that they are referred to as "chemical antibodies".

In the present invention, the "antibodies" may be produced by injection of the protein or are commercially available. In addition, examples of the antibodies include polyclonal antibodies, monoclonal antibodies, fragments capable of binding to epitopes, and the like.

Here, the polyclonal antibody may be produced by a conventional method of obtaining a serum containing the antibody by injecting the protein into an animal and collecting blood from the animal. This polyclonal antibody may be purified by any method known in the art, and may be produced from any animal species host, such as goat, rabbit, sheep, monkey, horse, pig, cow, dog, or the like.

In addition, the monoclonal antibody may be produced using any technique that provides for the production of antibody molecules through the culture of continuous cell lines. Such techniques include, but are not limited to, a hybridoma technique, a human B-cell line hybridoma technique, and an EBV-hybridoma technique.

In addition, antibody fragments containing a specific binding site for the above protein may be produced. For example, F(ab')2 fragments may be produced by pepsin digestion of antibody molecules, and Fab fragments may be produced by reducing disulfide bridges of F(ab')2 fragments, without being limited thereto. As an alternative method, a Fab expression library may be constructed at a small scale, thereby allowing rapid and/or easy identification of a monoclonal Fab fragment possessing a desired specificity.

In the present invention, the antibody may be bound to a solid substrate to facilitate subsequent processes such as washing or complex separation. Examples of the solid substrate include synthetic resins, nitrocellulose, glass substrates, metal substrates, glass fibers, microspheres, and microbeads. Also, examples of the synthetic resins include polyester, polyvinyl chloride, polystyrene, polypropylene, PVDF, and nylon.

In the present invention, the agent for reducing the expression level of the gene encoding the protein may comprise any one or more selected from the group consisting of antisense nucleotides, short interfering RNAs (siRNAs), short hairpin RNAs, and ribozymes, which bind complementarily to the gene encoding the protein, preferably the gene or a portion thereof, without being limited thereto. It corresponds to a means for providing an effect of inhibiting the expression of the gene encoding the target MRTF A protein by acting directly or indirectly on the gene encoding the target MRTF A protein, without being limited thereto, and may be any agent that can be easily obtained by a known technique commonly used in the art.

As an example of the present invention, the agent for reducing the expression level of the gene encoding the MRTF A protein may comprise any one or more selected from the group consisting of antisense nucleotides, short interfering RNA (siRNA), short hairpin RNA, and ribozymes, which bind complementarily to a polynucleotide consisting of a nucleotide sequence that is part or all of the gene encoding the MRTF A protein, without being limited thereto.

In the present invention, the "antisense nucleotides" bind (hybridize) to a complementary nucleotide sequence of DNA, immature-mRNA, or mature mRNA, as defined by Watson-Crick base pairing, and interrupt the flow of genetic information from DNA to protein. The specificity of antisense nucleotides to target sequences makes them exceptionally multi-functional. An antisense-nucleotide is a long chain of monomer units and thus may be readily synthesized to correspond to a target RNA sequence. Many reports have recently demonstrated the usefulness of antisense nucleotides as a biochemical tool in the study of target proteins. Many advances have been recently made in the fields of oligonucleotide chemistry and the synthesis of nucleotides having improved cell line adhesion, target binding affinity, and resistance to nucleases, suggesting that the use of antisense nucleotides may be considered a new type of inhibitor.

In the present invention, the "shRNA" and "siRNA" are nucleic acid molecules that can mediate RNA interference or gene silencing. They can inhibit the expression of target genes, and thus are used as an efficient gene knockdown method or gene therapy method. The shRNA forms a hairpin structure by binding between the complementary sequences in the single-strand oligonucleotide, and the shRNA *in vivo* may be cleaved by a dicer to become siRNA which is a double-stranded oligonucleotide as a small RNA fragment having a size of 21 to 25 nucleotides, and it may inhibit the expression of mRNA having a complementary sequence by binding specifically thereto. In addition, the siRNA refers to a small double-stranded RNA fragment of 21 to 25 nucleotides in size that modifies target mRNA by double-stranded RNA (dsRNA) to induce RNA interference (RNAi).

In the present invention, which means of the shRNA and siRNA is used may be determined by the selection of those skilled in the art, and the shRNA and siRNA may exhibit similar expression reduction effects if mRNA sequences targeted by them are the same as each other. For the purpose of the present invention, the shRNA or siRNA may act specifically on the gene encoding MRTF A and cleave the MRTF A gene (e.g., mRNA molecule) to induce RNA interference (RNAi), thereby inhibiting the expression of MRTF A. The siRNA may be chemically or enzymatically synthesized. A method of producing the siRNA is not particularly limited, and any method known in the art may be used. Examples of the method include, but are not limited to, a method of directly chemically synthesizing siRNA, a method of synthesizing siRNA using *in vitro* transcription, a method of enzymatically cleaving long double-stranded RNA synthesized by *in vitro* transcription, an expression method performed through intracellular delivery of an shRNA expression plasmid or viral vector, an expression method performed through intracellular delivery of a polymerase chain reaction (PCR)-induced siRNA expression cassette, and the like.

In the present invention, the term "ribozymes" refers to RNA molecules having catalytic activity. Ribozymes with various activities are known, and ribozymes for the MRTF A gene include known or artificially produced ribozymes, and optionally ribozymes having target-specific RNA cleavage activity may be produced by known standard techniques.

Another embodiment of the present invention is directed to a pharmaceutical composition for enhancing anticancer drug sensitivity.

In the present invention, the composition may contain a myocardin-related transcription factor A (MRTF A) inhibitor as an active ingredient.

In the present invention, the MRTF A inhibitor may comprise a compound represented by Formula 1 below or a pharmaceutically acceptable salt thereof: 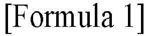 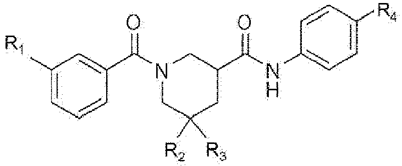 wherein
R₁ is a 5- or 6-membered ring heteroaryl, and R₂ to R₄ may each independently be hydrogen or halogen, wherein the heteroaryl may be furan or pyridine.

The MRTF A inhibitor of the present invention functions to inhibit Rho/MRTF/SRF signaling, and the compound represented by Formula 1 may be at least one selected from the group consisting of CCG-232601, CCG-203971, and CCG-222740, but is not limited thereto and may be any type of inhibitor that performs the same function.

In the present invention, the MRTF A inhibitor is preferably at least one selected from the group consisting of CCG-232601, CCG-203971, and CCG-222740. Details regarding the MRTF A inhibitor, CCG-232601, CCG-203971 and CCG-222740, the pharmaceutically acceptable salt, the anticancer drug, etc. are the same as those described above with respect to the pharmaceutical composition for treating anticancer drug resistance, and thus the description thereof will be omitted to avoid excessive complexity of the present specification.

The compounds represented by Formulas 1 to 4 according to the present invention are capable of effectively enhancing anticancer drug sensitivity. More specifically, these compounds may effectively increase the anticancer activity of anticancer drugs by increasing sensitivity to the anticancer drugs, indicating that they may be used very effectively for the prevention, alleviation or treatment of cancer.

In the present invention, the term "anticancer drug sensitivity" means the sensitivity of response to the use of an anticancer drug, and refers to a property that can maximize the effect of an anticancer drug on a subject upon administration of the anticancer drug. When the anticancer drug sensitivity is increased, it is possible to increase the therapeutic effect of the anticancer drug by allowing the effect of the drug to be exhibited smoothly.

In addition, the pharmaceutical composition for enhancing anticancer drug sensitivity according to the present invention may further contain an anticancer drug, thereby exhibiting a significantly improved effect of enhancing sensitivity. Here, although the anticancer drug that may be further contained is not particularly limited in the kind thereof, it may comprise, for example, at least one selected from the group consisting of cisplatin, nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, navelbine, fadrazole, tamoxifen, toremifene, testolactone, anastrozole, letrozole, vorozol, bicalutamide, lomustine, and carmustine. Preferably, the anticancer drug may be a platinum-based anticancer drug, and more preferably, may be at least one selected from the group consisting of cisplatin, carboplatin, and oxaliplatin.

Specifically, in the present invention, the compounds represented by Formulas 1 to 4, when co-administered with a general anticancer drug, more preferably a platinum-based anticancer drug, can enhance anticancer drug sensitivity, thereby providing a very high synergistic effect on inducing the inhibition of growth or apoptosis of cancer cells or cancer stem cells.

In one embodiment of the present invention, the compound represented by any one of Formulas 1 to 4 and the anticancer drug may be contained at a weight ratio of 1:0.01 to 100 or 1:0.1 to 400, without being limited thereto, and the content ratio may be appropriately adjusted depending on the kind of anticancer drug used. In addition, the compounds represented by Formulas 1 to 4 may be administered before or simultaneously with the use of the anticancer drug to which sensitivity is to be enhanced, and more preferably, may be administered 12 to 36 hours, most preferably 18 to 30 hours, before the use of the anticancer drug. If the content ratio is out of the above range, there may be problems in that the effect of enhancing anticancer drug sensitivity by co-administration cannot be obtained, and in that toxicity by the drug may occur in the subject of interest.

In another embodiment of the present invention, the compound represented by any one of Formulas 1 to 4 and the platinum-based anticancer drug may be contained at a weight ratio of 1:0.1 to 10, without being limited thereto. In addition, the compounds represented by Formulas 1 to 4 may be administered before or simultaneously with the use of the platinum-based anticancer drug to which sensitivity is to be enhanced, and more preferably, may be administered 12 to 36 hours, most preferably 18 to 30 hours, before the use of the platinum-based anticancer drug. If the content ratio is out of the above range, there may be problems in that the effect of enhancing anticancer drug sensitivity by co-administration cannot be obtained, and in that toxicity by the drug may occur in the subject of interest.

In another embodiment of the present invention, the compound represented by any one of Formulas 1 to 4 and cisplatin may be contained at a weight ratio of 1:0.1 to 10, without being limited thereto. In addition, the compounds represented by Formulas 1 to 4 may be administered before or simultaneously with the use of cisplatin to which sensitivity is to be enhanced, and more preferably, may be administered 12 to 36 hours, most preferably 18 to 30 hours, before the use of cisplatin. If the content ratio is out of the above range, there may be problems in that the effect of enhancing anticancer drug sensitivity by co-administration cannot be obtained, and in that toxicity by the drug may occur in the subject of interest.

In another embodiment of the present invention, the compound represented by any one of Formulas 1 to 4 and oxaliplatin may be contained at a weight ratio of 1:0.1 to 10, without being limited thereto. In addition, the compounds represented by Formulas 1 to 4 may be administered before or simultaneously with the use of oxaliplatin to which sensitivity is to be enhanced, and more preferably, may be administered 12 to 36 hours, most preferably 18 to 30 hours, before the use of oxaliplatin. If the content ratio is out of the above range, there may be problems in that the effect of enhancing anticancer drug sensitivity by co-administration cannot be obtained, and in that toxicity by the drug may occur in the subject of interest.

In addition, the composition of the present invention may contain, as an active ingredient, an agent for reducing the activity or expression level of myocardin-related transcription factor A (MRTF A) protein, or an agent for reducing the expression level of a gene encoding the protein.

Still another embodiment of the present invention is directed to a pharmaceutical composition for preventing or treating anticancer drug-resistant cancer.

In the present invention, the composition may contain a myocardin-related transcription factor A (MRTF A) inhibitor as an active ingredient.

In the present invention, the MRTF A inhibitor may comprise a compound represented by Formula 1 below or a pharmaceutically acceptable salt thereof: 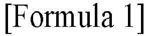 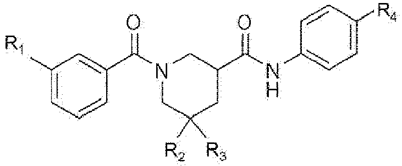 wherein
R₁ is a 5- or 6-membered ring heteroaryl, and R₂ to R₄ may each independently be hydrogen or halogen, wherein the heteroaryl may be furan or pyridine.

The MRTF A inhibitor of the present invention functions to inhibit Rho/MRTF/SRF signaling, and the compound represented by Formula 1 may be at least one selected from the group consisting of CCG-232601, CCG-203971, and CCG-222740, but is not limited thereto and may be any type of inhibitor that performs the same function.

In the present invention, the MRTF A inhibitor is preferably at least one selected from the group consisting of CCG-232601, CCG-203971, and CCG-222740. Details regarding the MRTF A inhibitor, CCG-232601, CCG-203971 and CCG-222740, the pharmaceutically acceptable salt, anticancer drug, etc. are the same as those described above with respect to the pharmaceutical composition for treating anticancer drug resistance, and thus the description thereof will be omitted to avoid excessive complexity of the present specification.

The compounds represented by Formulas 1 to 4 according to the present invention can very effectively treat anticancer drug-resistant cancer. The composition of the present invention may be used very effectively to prevent, ameliorate or treat cancer by lowering the anticancer drug resistance of stem-like malignant cancer and, at the same time, increasing the anticancer drug sensitivity of the cancer.

In addition, the pharmaceutical composition for preventing or treating anticancer drug-resistant cancer according to the present invention may further contain an anticancer drug, thereby significantly improving the efficiency of inhibiting the growth of cancer stem cells that induce anticancer drug resistance, thus exhibiting an improved effect of preventing or treating anticancer drug-resistant cancer.

Although the anticancer drug that is further contained is not particularly limited in the kind thereof, it may comprise, for example, at least one selected from the group consisting of cisplatin, nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, navelbine, fadrazole, tamoxifen, toremifene, testolactone, anastrozole, letrozole, vorozol, bicalutamide, lomustine, and carmustine. Preferably, the anticancer drug may be a platinum-based anticancer drug, and more preferably, may be at least one selected from the group consisting of cisplatin, carboplatin, and oxaliplatin.

Specifically, in the present invention, the compounds represented by Formulas 1 to 4, when co-administered with a general anticancer drug, more preferably a platinum-based anticancer drug can provide a very high synergistic effect on inducing the inhibition of growth or apoptosis of anticancer drug-resistant cancer cells or cancer stem cells.

In one embodiment of the present invention, the compound represented by any one of Formulas 1 to 4 and the anticancer drug may be contained at a weight ratio of 1:0.01 to 100 or 1:0.1 to 400, without being limited thereto, and the content ratio may be appropriately adjusted depending on the kind of anticancer drug used. In addition, the compounds represented by Formulas 1 to 4 may be administered before or simultaneously with the use of the anticancer drug, and more preferably, may be administered 12 to 36 hours, most preferably 18 to 30 hours, before the use of the anticancer drug. If the content ratio is out of the above range, there may be problems in that the effect of treating anticancer drug-resistant cancer by co-administration cannot be obtained, and in that toxicity by the drug may occur in the subject of interest.

In another embodiment of the present invention, the compound represented by any one of Formulas 1 to 4 and the platinum-based anticancer drug may be contained at a weight ratio of 1:0.1 to 10, without being limited thereto. In addition, the compounds represented by Formulas 1 to 4 may be administered before or simultaneously with the use of the platinum-based anticancer drug, and more preferably, may be administered 12 to 36 hours, most preferably 18 to 30 hours, before the use of the platinum-based anticancer drug. If the content ratio is out of the above range, there may be problems in that a desired effect of treating anticancer drug-resistant cancer by co-administration cannot be obtained, and in that toxicity by the drug may occur in the subject of interest.

In another embodiment of the present invention, the compound represented by any one of Formulas 1 to 4 and cisplatin may be contained at a weight ratio of 1:0.1 to 10, without being limited thereto. In addition, the compounds represented by Formulas 1 to 4 may be administered before or simultaneously with the use of cisplatin, and more preferably, may be administered 12 to 36 hours, most preferably 18 to 30 hours, before the use of cisplatin. If the content ratio is out of the above range, there may be problems in that a desired effect of treating anticancer drug-resistant cancer by co-administration cannot be obtained, and in that toxicity by the drug may occur in the subject of interest.

In another embodiment of the present invention, the compound represented by any one of Formulas 1 to 4 and oxaliplatin may be contained at a weight ratio of 1:0.1 to 10, without being limited thereto. In addition, the compounds represented by Formulas 1 to 4 may be administered before or simultaneously with the use of oxaliplatin to which resistance is to be overcome, and more preferably, may be administered 12 to 36 hours, most preferably 18 to 30 hours, before the use of the oxaliplatin. If the content ratio is out of the above range, there may be problems in that a desired effect of overcoming anticancer drug resistance by co-administration cannot be obtained, and in that toxicity by the drug may occur in the subject of interest.

In addition, the composition of the present invention may also contain, as an active ingredient, an agent for reducing the activity or expression level of myocardin-related transcription factor A (MRTF A) protein, or an agent for reducing the expression level of a gene encoding the protein.

The disease to be prevented, alleviated, or treated by the composition of the present invention may be an anticancer agent-resistant cancer that has developed or is likely to develop in a subject of interest.

In the present invention, the term "subject of interest" refers to mammals, including humans. For example, the subject of interest may be selected from the group consisting of humans, rats, mice, guinea pigs, hamsters, rabbits, monkeys, dogs, cats, cows, horses, pigs, sheep, and goats, and preferably may be a human, without being limited thereto.

In the present invention, the term "human" may refer to a person who has or is suspected of having cancer, and may mean a patient who needs or is expected to receive appropriate cancer treatment, without being limited thereto.

In the present invention, the term "preventing" may include, without limitation, any action that blocks, inhibits or delays symptoms, which are caused by the uncontrolled growth of cancer cells, by using the pharmaceutical composition of the present invention.

In the present invention, the term "alleviating" may include, without limitation, any action that alleviates or beneficially changes symptoms, which are caused by the uncontrolled growth of cancer cells, by using the pharmaceutical composition of the present invention.

In the present invention, the term "treating" may include, without limitation, any action that alleviates or beneficially changes symptoms caused by the uncontrolled growth of cancer cells by using the pharmaceutical composition of the present invention.

In the present invention, the pharmaceutical composition may be in the form of capsules, tablets, granules, injections, ointments, powders, or beverages, and the pharmaceutical composition may be for administration to humans.

For use, the pharmaceutical composition of the present invention may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition of the present invention may further contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used for injection; and a base, an excipient, a lubricant, a preservative, and the like may be used for topical administration. The pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further contain a filler, an anticoagulant, a lubricant, a wetting agent, a fragrance, an emulsifier, a preservative, or the like.

The routes of administration of the pharmaceutical composition according to the present invention include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intra-lesional and intra-cranial injection or infusion techniques. The pharmaceutical composition of the present invention may also be formulated as suppositories for intrarectal administration.

The dose of the pharmaceutical composition of the present invention may vary depending on various factors, including the activity of a specific compound used, the patient's age, body weight, general health status, sex, and diet, the time of administration, the route of administration, excretion rate, drug combination, and the severity of a particular disease to be prevented or treated. Although the dose of the pharmaceutical composition may vary depending on the patient's condition and body weight, the severity of the disease, the form of drug, and the route and duration of administration, it may be appropriately selected by those skilled in the art. The pharmaceutical composition may be administered at a dose of 0.0001 to 50 mg/kg/day or 0.001 to 50 mg/kg/day. The pharmaceutical composition may be administered once a day or several times a day. The dose does not limit the scope of the present invention in any way. The pharmaceutical composition according to the present invention may be formulated as pills, sugar-coated tablets, capsules, liquids, gels, syrups, slurries, or suspensions.

Yet another embodiment of the present invention is directed to a method for treating anticancer drug resistance or enhancing anticancer drug sensitivity.

As an example, the method of the present invention may comprise a step of administering an effective amount of a myocardin-related transcription factor A (MRTF A) inhibitor to a subject in need thereof.

As another example, the method of the present invention may comprise steps of administering to a subject thereof an effective amount of an agent for reducing the activity or expression level of MRTF A protein, or an agent for reducing the expression level of a gene encoding the protein.

Yet another embodiment of the present invention is directed to a method for treating anticancer drug resistance or enhancing anticancer drug sensitivity.

As an example, the method of the present invention may comprise a step of administering an effective amount of a myocardin-related transcription factor A (MRTF A) inhibitor to a subject in need thereof.

As another example, the method of the present invention may comprise steps of administering to a subject thereof an effective amount of an agent for reducing the activity or expression level of MRTF A protein, or an agent for reducing the expression level of a gene encoding the protein.

Yet another embodiment of the present invention is directed to a method for treating anticancer drug resistance or enhancing anticancer drug sensitivity.

As an example, the method of the present invention may comprise a step of administering an effective amount of a myocardin-related transcription factor A (MRTF A) inhibitor to a subject in need thereof.

As another example, the method of the present invention may comprise steps of administering to a subject thereof an effective amount of an agent for reducing the activity or expression level of MRTF A protein, or an agent for reducing the expression level of a gene encoding the protein.

In the method for treating anticancer drug resistance according to the present invention, the method for enhancing anticancer drug sensitivity, and the method for preventing or treating anticancer drug-resistant cancer, details regarding the MRTF A inhibitor, each protein or the gene encoding the same, the agent for reducing the activity or expression of the protein, the agent for reducing the expression level of the gene encoding the protein, and the like are the same as those described above with respect to the composition for overcoming anticancer drug resistance, and thus the description thereof will be omitted to avoid excessive complexity of the present specification.

In the present invention, the term "administering" means providing a given composition of the present invention to a subject by any suitable method.

In the present invention, the phrase "subject in need thereof" may include both mammals and non-mammals. Here, examples of the mammals include, but are not limited to, humans, non-human primates such as chimpanzees, other ape or monkey species; livestock animals such as cattle, horses, sheep, goats, and pigs; domesticated animals such as rabbits, dogs or cats; laboratory animals, for example, rodents such as rats, mice, or guinea pigs. In addition, examples of the non-mammals in the present invention include, but are not limited to, birds or fish.

In the present invention, the formulation of the composition that is administered as described above is not particularly limited, and may be administered as solid form preparations, liquid form preparations, or preparations for inhalation. Specifically, the composition may be administered as solid form preparations which are intended to be converted, shortly before use, to liquid form preparations for oral or parenteral administration. For example, the composition may be formulated and administered as oral dosage forms, including powders, granules, capsules, tablets, and aqueous suspensions, preparations for external use, suppositories, and sterile injectable solutions, without being limited thereto.

In addition, in the present invention, pharmaceutically acceptable carriers may be additionally administered together with the composition of the present invention. Here, as the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used for injection; and a base, an excipient, a lubricant, a preserving agent, and the like may be used for topical administration. In addition, the compound of the present invention may be prepared in various dosage forms by being mixed with the pharmaceutically acceptable carriers as described above. For example, for oral administration, the compound may be formulated in the form of tablets, troches, capsules, elixirs, suspensions, syrups, wafers, or the like. For injection, the compound may be formulated in the form of unit dosage ampoules or in multiple-dosage forms. In addition, the compound may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the composition of the present invention may further contain a filler, an anticoagulant, a lubricant, a wetting agent, a fragrance, an emulsifier, a preservative, or the like.

The routes of administration of the composition according to the present invention include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, the term "parenteral" includes subcutaneous, transdermal, intravenous, intramuscular, intra-articular, intra-synovial, intrasternal, intradural, intra-lesional and intra-cranial injection or infusion techniques. The pharmaceutical composition of the present invention may also be formulated as suppositories for intrarectal administration.

In the present invention, the term "pharmaceutically effective amount" refers to a sufficient amount of an agent to provide a desired biological result. Said result may be reduction and/or alleviation of a sign, symptom, or cause of a disease, or any other desired alteration of a biological system. For example, an "effective amount" for therapeutic uses is the amount of the composition disclosed in the present invention, which is required to provide a clinically significant reduction in the disease. An appropriate "effective amount" in any individual case may be determined by one of ordinary skill in the art using routine experimentation. Thus, the expression "effective amount" generally refers to an amount in which an active substance has a therapeutic effect. In the case of the present invention, the active substance serves as both an agent for treating anticancer drug resistance and an agent for preventing, alleviating or treating anticancer drug-resistant cancer.

The dose of the composition of the present invention may vary depending on various factors, including the activity of an active substance used, the patient's age, body weight, general health status, sex, and diet, the time of administration, the route of administration, excretion rate, drug combination, and the severity of a particular disease to be prevented or treated. Although the dose of the active substance may vary depending on the patient's condition and body weight, the severity of the disease, the form of drug, and the route and duration of administration, it may be appropriately selected by those skilled in the art. The active substance may be administered at a dose of 0.0001 to 100 mg/kg/day or 0.001 to 100 mg/kg/day. The active substance may be administered once a day or several times a day. The dose does not limit the scope of the present invention in any way. The compound according to the present invention may be formulated as pills, sugar-coated tablets, capsules, liquids, gels, syrups, slurries, or suspensions.

The active substance of the present invention may be used alone or in combination with surgery, radiotherapy, hormone therapy, chemotherapy, and methods that use biological response modifiers.

In addition, the composition of the present invention may also be used in combination with an anticancer drug. Here, the anticancer drug may be, but is not limited to, at least one selected from the group consisting of nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, sorafenib, bevacizumab, cisplatin, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, capecitabine, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, olaparib, navelbine, fadrazole, tamoxifen, toremifene, testolactone, anastrozole, letrozole, vorozole, bicalutamide, lomustine, vorinostat, entinostat, phenformin, metformin, talazoparib, and carmustine.

Still yet another embodiment of the present invention is directed to a method for screening a composition for treating anticancer drug resistance.

The method of the present invention may comprise steps of: bringing a candidate substance into contact with a biological sample containing myocardin-related transcription factor A (MRTF A) protein or a cell expressing the same; and measuring the activity or expression level of the MRTF A protein or a gene encoding the same.

In one embodiment of the present invention, when the measured activity or expression level of the MRTF A protein or the gene encoding the same decreased, the candidate substance may be determined as a composition for treating anticancer drug resistance.

In the present invention, the term "screening" means selecting a desired substance having any specific property from a candidate group consisting of a plurality of substances by a specific manipulation or evaluation method.

In the present invention, the cell may be engineered to overexpress the MRTF A protein or the gene encoding the same, and preferably, may be transfected by introduction of a recombinant vector containing the gene encoding the protein into the cell.

### Advantageous Effects

The composition according to the present invention, when used alone, is capable of inhibiting the growth of anticancer drug-resistant cancer by overcoming anticancer drug resistance or enhancing anticancer drug sensitivity, and when co-administered with anticancer drugs, may be used very effectively for the prevention, alleviation or treatment of various cancers, particularly anticancer drug-resistant cancer.

### Brief Description of Drawings

FIG. 1 shows the results of evaluating the survival and growth inhibitory effects upon treatment of cancer cell lines and cell lines having cancer stem cell characteristics with cisplatin (CDDP) or CCG-232601 by a cell viability measurement method according to one example of the present invention.
FIG. 2 shows the results of measuring the oxygen consumption rate (OCR) and extracellular acidification rate (ECAR) of cells after treating HS746T and MKN1 cell lines having cancer stem cell characteristics with CCG-232601 according to one example of the present invention.
FIG. 3 shows the results of evaluating the cancer metastasis inhibitory effect upon treatment of HS746T and MKN1 cell lines having cancer stem cell characteristics with CCG-232601 according to one example of the present invention.
FIG. 4 shows the results of evaluating the drug sensitivity upon treatment of HS746T and MKN1 cell lines with cisplatin according to one example of the present invention.
FIG. 5 is a schematic view showing a process of co-treating the MKN1 cell line with CCG-232601 and cisplatin according to one example of the present invention.
FIG. 6 shows the results of evaluating the survival and growth inhibitory effects upon treatment of the MKN1 cell line with CCG-232601 and cisplatin alone or in combination with each other by using a cell viability assay kit (Cell Counting Kit-8; CCK-8) according to one example of the present invention.
FIG. 7 shows the results of evaluating the survival and growth inhibitory effects upon treatment of the HS746T cell line with CCG-232601 and oxaliplatin alone or in combination with each other by using a cell viability assay kit (Cell Counting Kit-8; CCK-8) according to one example of the present invention.
FIG. 8 shows the results of evaluating the cisplatin resistance treatment effect upon treatment of the HS746T cell line with CCG-232601, CCG-203971 or CCG-222740 in combination with cisplatin by using a cell viability assay kit (Cell Counting Kit-8; CCK-8) according to one example of the present invention.

### Best Mode

One embodiment of the present invention is directed to a pharmaceutical composition for treating anticancer drug resistance containing a myocardin-related transcription factor A (MRTF A) inhibitor.

Another embodiment of the present invention is directed to a pharmaceutical composition for enhancing anticancer drug sensitivity containing an MRTF A inhibitor.

Still another embodiment of the present invention is directed to a method for treating anticancer drug resistance or enhancing anticancer drug sensitivity comprising a step of administering an effective amount of an MRTF A inhibitor to a subject in need thereof.

Yet another embodiment of the present invention is directed to a pharmaceutical composition for preventing or treating anticancer drug-resistant cancer containing an MRTF A inhibitor.

Still yet another embodiment of the present invention is directed to a method for preventing or treating anticancer drug-resistant cancer comprising a step of administering an effective amount of an MRTF A inhibitor to a subject in need thereof.

A further embodiment of the present invention is directed to a method for screening a drug for overcoming or treating anticancer drug resistance or a drug for enhancing anticancer drug sensitivity, the method comprising steps of: bringing a candidate substance into contact with a biological sample containing myocardin-related transcription factor A (MRTF A) protein or a cell expressing the same; and measuring the activity or expression level of the MRTF A protein or a gene encoding the same after treatment with the candidate substance.

### Mode for Invention

Hereinafter, the present invention will be described in more detail with reference to examples. These examples are only for explaining the present invention in more detail, and it will be apparent to those skilled in the art that the scope of the present invention according to the subject matter of the present invention is not limited by these examples.

### Preparation Example: Culture of cell lines

In order to evaluate the ability to inhibit the growth or metastasis of cancer cell lines or cancer stem cell lines in Examples of the present invention, the present inventors obtained the cell lines MKN1 and HS746T having human gastric cancer stem cell characteristics, and the human gastric cancer cell lines SNU601, YCC7 and NCIN87, from the American Type Culture Collection (ATCC) and the Korean Cell Line Bank (KCRB). The cell lines were cultured in different culture media under different culture conditions according to the ATCC or KCRB guides. The MKN1, SNU601, and NCIN87 cell lines were cultured in RPMI1640 media containing 10% fetal bovine serum (FBS), 2 mM L-glutamine, 100 U/ml penicillin and 100 µg/ml streptomycin, and the HS746T and YCC7 cell lines were cultured in DMEM media containing 10% FBS, 2 mM L-glutamine, 100 U/ml penicillin and 100 µg/ml streptomycin. All the cell lines were cultured at 37°C under 5% CO₂, and mycoplasma contamination tests were performed.

### Example 1: Evaluation of therapeutic effect against cancer cells and cancer stem cells upon treatment with CCG-232601

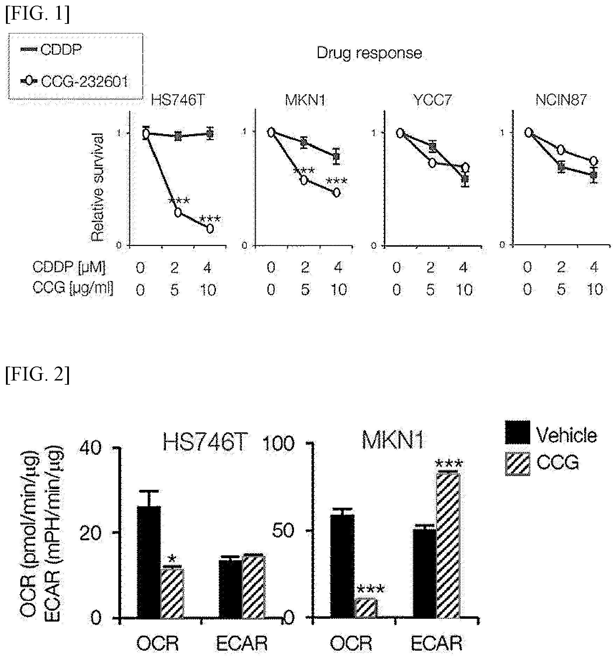

The cell lines cultured in the Preparation Example were seeded into well plates at a density of 1 × 10⁴ cells per well, and cultured overnight at 37°C under 5% CO₂. Next, each of the gastric cancer stem-like cell lines HS746T and MKN1 and the gastric cancer cell lines YCC7 and NCIN87 was treated with different concentrations of each of the anticancer drug cisplatin (CDDP), which is currently widely used in the treatment of gastric cancer patients, and the MRTF A inhibitor CCG-232601 compound, and after 48 hours, the number of cells was counted and the results are shown in FIG. 1. 100 µL of each cell culture and 10 µL of reaction buffer (CCK) were mixed together, placed in a 96-well plate, incubated for 1 hour at room temperature, and then measured for absorbance at a wavelength of 450 nm using a spectrophotometer (Synergy HTX Multi-Reader, BioTek , Winooski, VT, USA). At the same time, the number of cells in each sample was counted using a cell viability assay kit (Cell Counting Kit-8), and the counted value was expressed relative to the cell number of the negative control group so that the same cell number in each cell could be compared with the cell number of the negative control group. All experiments were repeated 3 times and the results are shown in FIG. 1. The experimental results are shown as the mean values of the repeated experiments.

The experimental results indicated that treatment with the CCG-232601 compound alone exhibited antitumor effects not only on the cancer cell lines but also on the cancer stem cell lines, and that treatment with cisplatin alone exhibited significantly improved antitumor effects in the stem-like cell lines HS746T and MKN1 against which antitumor effects were previously not exhibited due to their drug resistance (see FIG. 1). As such, it can be seen that the CCG-232601 compound according to the present invention can reduce the viability of cancer cells and cancer stem cells and inhibit the growth thereof.

In particular, in order to confirm that the CCG-232601 compound has an excellent inhibitory effect against the growth of cancer stem cells, the oxygen consumption rate (OCR) and extracellular acidification rate (ECAR) of the stem-like cell lines HS746T and MKN1 having high resistance to metabolic stress were measured and the results are FIG. 2. More specifically, each of the cell lines was inoculated at a confluency of 70 to 80% in Seahorse normal culture medium, and oxygen consumption rate (OCR, pmol/min/µg protein) and extracellular acidification rate (ECAR, mpH/min/µg protein) were analyzed using an XF96 or XFp extracellular flux analyzer (Agilent) to determine mitochondrial oxidation and glycolysis levels. Mitochondrial oxidation ability of the cells pretreated for 1 hour with or without 10 mM β-HB (Sigma) was evaluated using the Seahorse XF Mito Stress Kit (Agilent). To overcome changes in oxidation and glycolysis processes upon treatment with CCG-232601, the cells were pretreated overnight with vehicle or the inhibitor, and finally 10 mM β-hydroxybutyrate was added upon the first injection.

As a result of the experiment, it was shown that, upon treatment with CCG-232601, the glycolytic ECAR was higher than the OCR, which is an indicator of mitochondrial respiration (see FIG. 2).

From the above results, it was confirmed that treatment with the CCG-232601 compound greatly increased the susceptibility of the gastric cancer cells, which have resistance to metabolic stress as a representative characteristic, to glucose deprivation.

### Example 2: Evaluation of cancer metastasis inhibitory effect upon administration of CCG-232601

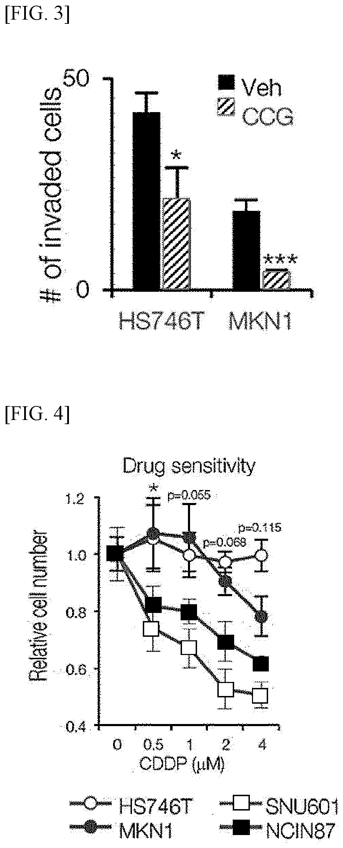

Transwell invasion assay was performed with or without CCG-232601 treatment. Invasion ability was measured using a 6.5-mm transwell with an 8.0 µm-pore polycarbonate membrane insert (Corning) after coating with matrigel (Corning) diluted with serum-free medium to 0.67 µg/µl. Then, 2 × 10⁴ cells per well were suspended in 200 µl of serum-free medium with vehicle or 10 µg/ml CCG-232601, and 1,000 µl of culture medium containing 10% FBS was added to the lower chamber. After 12 hours, the transferred cells were stained with 0.2% crystal violet and observed under an optical microscope. The average number of cells penetrating the membrane was counted with ImageJ (NIH) in three randomly selected high-power fields and two independent experiments, and the results are shown in FIG. 3.

As a result of the experiment, it was shown that treatment with the MRTFA/SRF inhibitor CCG-232601 reduced the invasion and metastasis of the HS746T and MKN1 cell lines.

From the above results, it can be seen that the compound according to the present invention can very effectively inhibit cancer cell invasion and cancer metastasis by inhibiting MRTFA/SRF of cancer cells.

### Example 3: Evaluation (1) of drug sensitivity improvement effect of MRTF A inhibitor

Drug sensitivity upon treatment with cisplatin (CDDP) was measured in the gastric cancer cell lines SNU601 and NCIN87 and the cell lines HS746T and MKN1 having gastric cancer stem cell-like characteristics, and the results are shown in FIG. 3. As a result of the experiment, it was shown that the cancer stem-like cell lines HS746T and MKN1 showed resistance to cisplatin, and that the drug sensitivity of the cancer stem cell lines was about 20 to 40% lower than that of the cancer cell lines (see FIG. 4).

### 3-1. Cisplatin resistance treatment effect of compound CCG-232601

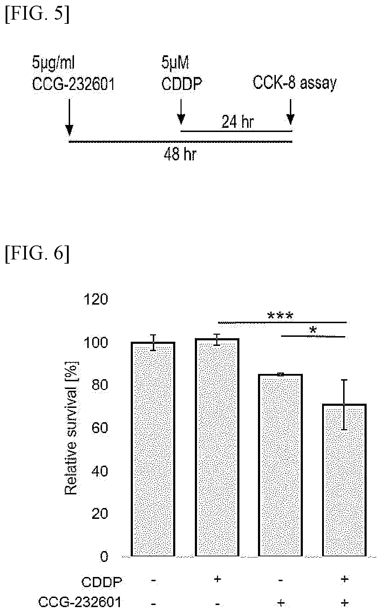

Accordingly, in order to evaluate the effect of co-administration of the compound CCG-232601, the survival and growth inhibitory effects upon administration of cisplatin (CDDP) and CCG-232601 alone or in combination to the MKN1 cell line were evaluated using a cell viability assay kit (Cell Counting Kit- 8; CCK-8). A schematic view of a specific experimental process for this evaluation is shown in FIG. 5. Unlike administration alone, to evaluate the effect of co-administration, 5 µg/ml CCG-232601 was first administered to the MKN1 cell line, and 24 hours after administration of CCG-232601, 5 µM cisplatin (CDDP) was administered to the cell line, and 24 hours after administration of CDDP, CCK-8 assay was performed using a cell viability assay kit.

As a result of the experiment, it was shown that, when the compound CCG-232601 according to the present invention was administered alone, the cell viability of the MKN1 cell line having cancer stem cell characteristics was reduced by 20% compared to the negative control group and the group to which cisplatin was administered alone. In addition, it could be seen that the drug resistance that appeared when cisplatin was administered alone was overcome when the cell line was treated with cisplatin in combination with the compound CCG-232601 (see FIG. 6).

### 3-2. Oxaliplatin resistance treatment effect of compound CCG-232601

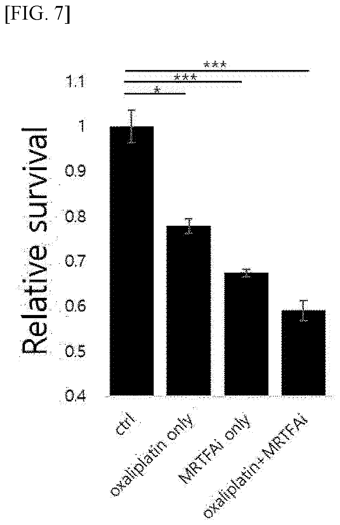

In order to examine whether the co-administration of the compound CCG-232601 is also effective in improving drug sensitivity to other platinum-based anticancer drugs of the same family as cisplatin, the survival and growth inhibitory effects upon administration of CCG-232601 and oxaliplatin alone or in combination to the HS746T cell line were evaluated using a cell viability assay kit (Cell Counting Kit-8; CCK-8). A schematic view of a specific experimental process for this evaluation is shown in FIG. 7. Unlike administration alone, to evaluate the effect of co-administration, 5 µg/ml CCG-232601 was first administered to the HS746T cell line, and 24 hours after administration of CCG-232601, 24 µg/µl oxaliplatin was administered to the cell line, and 24 hours after administration of oxaliplatin, CCK-8 assay was performed using a cell viability assay kit.

As a result of the experiment, it was shown that, when the compound CCG-232601 according to the present invention was administered alone, it reduced the growth of the cancer stem-like cells by itself, and that, when the compound CCG-232601 was administered in combination with oxaliplatin, the cell viability was reduced by about 20% or more compared to when oxaliplatin was administered alone, suggesting that the co-administration had the effect of significantly improving drug sensitivity (see FIG. 7).

### Example 4: Evaluation (2) of drug sensitivity improvement effect of MRTF A inhibitor

In order to evaluate and verify the anticancer drug resistance treatment effect upon co-administration of various MRTF A inhibitors, including the compound CCG-232601, the survival and growth inhibitory effects upon administration of CCG-232601, CCG-203971 or CCG-222740 and cisplatin (CDDP) alone or in combination to the HS746T cell line were evaluated using a cell viability assay kit (Cell Counting Kit-8; CCK-8). In the same manner as described above, 5 µg/ml CCG-232601, CCG-203971 or CCG-222740 was first administered to the HS746T cell line, and after 24 hours, 5 µM cisplatin (CDDP) was administered to the cell line, and 24 hours after administration of CDDP, a CCK-8 assay was performed using a cell viability assay kit.

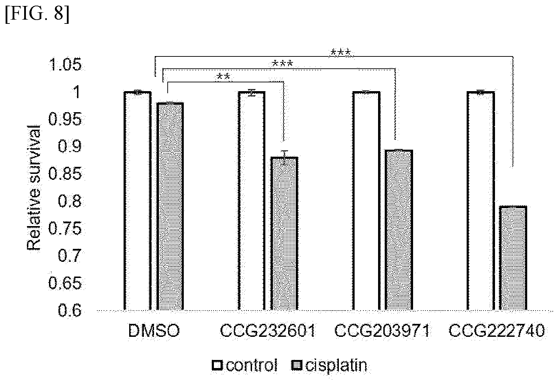

As a result of the experiment, it was could be seen that drug sensitivity was improved when cisplatin was administered in combination with the MRTF A inhibitor (CCG-232601, CCG-203971 or CCG-222740) compared to when cisplatin was administered alone (see FIG. 8).

Taking the above results together, it can be seen that the MRTF A inhibitor according to the present invention has an effect of improving sensitivity to anticancer drugs, particularly platinum-based anticancer drugs, and thus may provide a synergistic effect on inhibiting the growth of cancer stem cells and also overcome resistance to platinum-based anticancer drugs.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The composition according to the present invention may overcome anticancer drug resistance, and at the same time, enhance anticancer drug sensitivity, and furthermore, effectively prevent, alleviate or treat anticancer drug-resistant cancer.

## Claims

1. A pharmaceutical composition for treating anticancer drug resistance containing a myocardin-related transcription factor A (MRTF A) inhibitor.

2. The composition according to claim 1, wherein the MRTF A inhibitor is a compound represented by Formula 1 below or a pharmaceutically acceptable salt thereof: 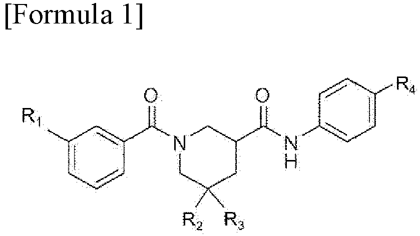 wherein
R₁ is a 5- or 6-membered ring heteroaryl, and R₂ to R₄ are each independently hydrogen or halogen.

3. The composition according to claim 2, wherein the compound represented by Formula 1 corresponds to at least one selected from the group consisting of compounds represented by Formulas 2 to 4 below:

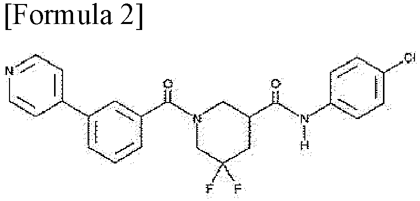

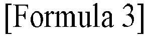

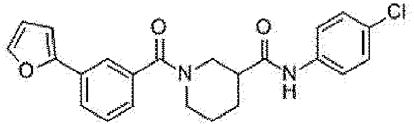

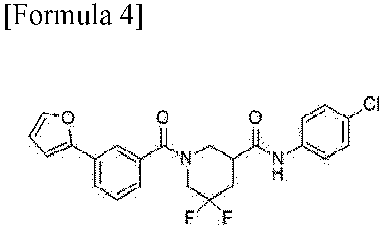

4. The composition according to claim 1, wherein the anticancer drug is at least one selected from the group consisting of cisplatin, carboplatin, and oxaliplatin.

5. The composition according to claim 1, further containing an anticancer drug.

6. The composition according to claim 5, wherein the anticancer drug is at least one selected from the group consisting of cisplatin, nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, navelbine, fadrazole, tamoxifen, toremifene, testolactone, anastrozole, letrozole, vorozol, bicalutamide, lomustine, and carmustine.

7. The composition according to claim 6, wherein the anticancer drug is at least one selected from the group consisting of cisplatin, carboplatin, and oxaliplatin, which are platinum-based anticancer drugs.

8. The composition according to claim 7, wherein the anticancer drug is cisplatin.

9. A pharmaceutical composition for enhancing anticancer drug sensitivity containing a myocardin-related transcription factor A (MRTF A) inhibitor.

10. The composition according to claim 9, wherein the MRTF A inhibitor is a compound represented by Formula 1 below or a pharmaceutically acceptable salt thereof: 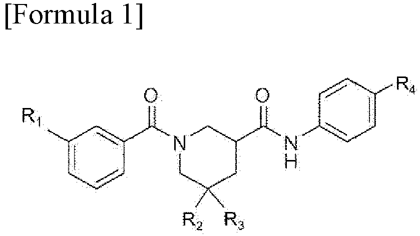 wherein
R₁ is a 5- or 6-membered ring heteroaryl, and R₂ to R₄ are each independently hydrogen or halogen.

11. The composition according to claim 10, wherein the compound represented by Formula 1 corresponds to at least one selected from the group consisting of compounds represented by Formulas 2 to 4 below:

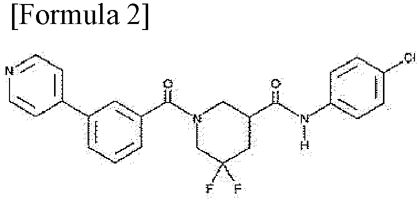

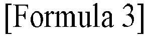

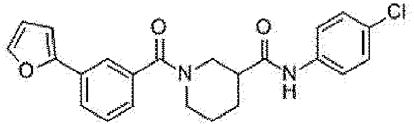

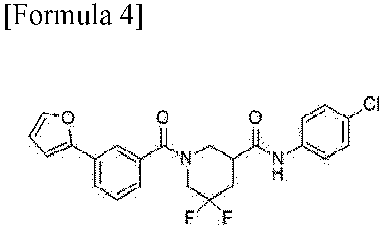

12. The composition according to claim 9, wherein the anticancer drug is at least one selected from the group consisting of cisplatin, carboplatin, and oxaliplatin.

13. The composition according to claim 9, further containing an anticancer drug.

14. The composition according to claim 13, wherein the anticancer drug is at least one selected from the group consisting of cisplatin, nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, navelbine, fadrazole, tamoxifen, toremifene, testolactone, anastrozole, letrozole, vorozol, bicalutamide, lomustine, and carmustine.

15. The composition according to claim 14, wherein the anticancer drug is at least one selected from the group consisting of cisplatin, carboplatin, and oxaliplatin, which are platinum-based anticancer drugs.

16. The composition according to claim 15, wherein the anticancer drug is cisplatin.

17. A pharmaceutical composition for preventing or treating anticancer drug-resistant cancer containing a myocardin-related transcription factor A (MRTF A) inhibitor.

18. The composition according to claim 17, wherein the MRTF A inhibitor is a compound represented by Formula 1 below or a pharmaceutically acceptable salt thereof: 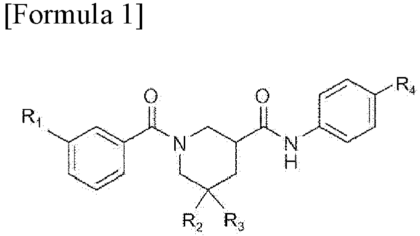 wherein
R₁ is a 5- or 6-membered ring heteroaryl, and R₂ to R₄ are each independently hydrogen or halogen.

19. The composition according to claim 18, wherein the compound represented by Formula 1 corresponds to at least one selected from the group consisting of compounds represented by Formulas 2 to 4 below:

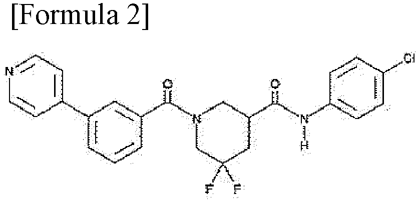

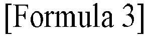

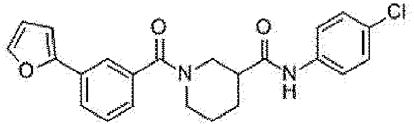

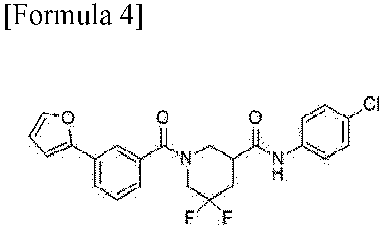

20. The composition according to claim 17, further containing an anticancer drug.

21. The composition according to claim 20, wherein the anticancer drug is at least one selected from the group consisting of cisplatin, nitrogen mustard, imatinib, oxaliplatin, rituximab, erlotinib, neratinib, lapatinib, gefitinib, vandetanib, nirotinib, semasanib, bosutinib, axitinib, cediranib, lestaurtinib, trastuzumab, gefitinib, bortezomib, sunitinib, carboplatin, bevacizumab, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, fluorouracil, fludarabine, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, temozolomide, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, navelbine, fadrazole, tamoxifen, toremifene, testolactone, anastrozole, letrozole, vorozol, bicalutamide, lomustine, and carmustine.

22. The composition according to claim 17, wherein the cancer is at least one selected from the group consisting of gastric cancer, breast cancer, colorectal cancer, lung cancer, liver cancer, esophageal cancer, pancreatic cancer, gallbladder cancer, kidney cancer, bladder cancer, prostate cancer, testicular cancer, colon cancer, cervical cancer, endometrial cancer, choriocarcinoma, skin cancer, ovarian cancer, thyroid cancer, brain cancer, blood cancer, head and neck cancer, malignant melanoma, and lymphoma.

23. A method for screening a composition for treating anticancer drug resistance, the method comprising steps of:
bringing a candidate substance into contact with a biological sample containing myocardin-related transcription factor A (MRTF A) protein or a cell expressing the same; and
measuring the activity or expression level of the MRTF A protein or a gene encoding the same, wherein, when the measured activity or expression level of the MRTF A protein or the gene encoding the same decreased, the candidate substance is determined as a composition for treating anticancer drug resistance.
